# EUROPÄISCHE PATENTSCHRIFT

(11) **EP 2 979 668 B1**
(45) Veröffentlichungstag und Bekanntmachung des Hinweises auf die Patenterteilung: **07.11.2018**
(21) Anmeldenummer: 14002643.6
(22) Anmeldetag: 29.07.2014
(51) Int. Cl.: A61F 5/11

(54) **Befestigungsvorrichtung für Nagelkorrekturspangen**
Fastening device for nail correcting braces
Dispositif de fixation pour agrafes de correction d'ongle

(43) Veröffentlichungstag der Anmeldung: 03.02.2016
(73) Patentinhaber: 3TO GmbH, 82041 Deisenhofen (DE)
(72) Erfinder: Sutor, Norbert, D-82041 Deisenhofen (DE); Sutor, Franziska, D-80333 München (DE); Sutor, Johannes, D-80339 München (DE)
(74) Vertreter: Müller, Bernhard

(56) Entgegenhaltungen:
- EP-A1- 2 158 881
- WO-A2-2007/102156
- DE-U1- 8 136 484
- FR-A1- 2 939 638

## Beschreibung

Die Erfindung betrifft eine Befestigungsvorrichtung für Nagelkorrekturspangen gemäß Oberbegriff des Hauptanspruchs.

Zur Behandlung von eingewachsenen oder eingerollten Nägeln, wurden verschiedene Nagelkorrekturspangensysteme entwickelt. Diese so genannten Orthonyxiespangen reduzieren die Krümmung der Nagelplatte und entlasten dadurch den oftmals entzündeten Nagelfalz. Die Schmerzen werden deutlich gemindert und eine Abheilung ermöglicht.

Als sehr effizient haben sich Nagelkorrekturspangen bewährt, deren Wirkung auf Rückstellkräften von Federelementen, oft in Form von Metalldrähten, die unter den Nagelrand eingehängt und/oder auf die Nageloberfläche aufgeklebt werden, beruht.

Schon vor Jahrzehnten wurde die sogenannte Fraserspange entwickelt, eine einteilige Drahtspange, welche beidseitig unter den Nagel greift und diesen mittels Rückstellkraft anhebt. Da bei dieser einteiligen Spange jedoch eine exakte Anpassung auf die Nagelbreite notwendig ist und sie von der Nagelvorderseite aufgeschoben werden muss, haben sich zunehmend mehrteilige Spangensysteme etabliert. So wurde erstmals von Günther Greppmayr in der Ausgabe März 1968 der Fachzeitschrift "Der FUSS" eine zweiteilige Ausführung dieser Fraserspange beschrieben. Hier wird ein einseitig unter den Nagelrand eingehängter Draht auf der anderen Nagelseite in ein auf die Nageloberfläche aufgeklebtes Drahtgewebe mit einzelnen herausgezogenen Maschen eingeführt.

Als Weiterentwicklung des beschriebenen Systems sind in der DE 10 2008 039 762 A1 verschiedene Befestigungseinrichtungen für unter den Nagel greifende Nagelkorrekturdrähte beschrieben.

Eine Vorrichtung zur Nagelkorrektur bestehend aus zwei, auf den Nagel aufgeklebten Befestigungseinrichtungen, welche durch ein loses Federelement miteinander verbunden werden, ist in der DE 3330813 A1 beschrieben. Hier werden die Elemente zur Krafteinleitung auf die Nagelplatte geklebt, die Spangenkraft wird anschließend über ein einzuschiebendes Federelement aufgebracht.

Bei allen bisher bekannten Befestigungsvorrichtungen für Nagelkorrekturspangen bereitet das Einführen des entsprechenden Federelementes in die hierfür vorgesehenen Hinterschneidungen auf Grund der filigranen Abmessungen meist größere Schwierigkeiten.

Nachteilig ist auch, dass bei den meisten Einrichtungen ein mehrfaches Herausspringen des Nagelkorrekturdrahtes aus der Befestigungsvorrichtung während der Montage in Kauf genommen werden muss. Zudem muss beim Aufkleben der Befestigungselemente auf den Nagel genau auf die Ausrichtung im richtigen Winkel zu den jeweiligen Federelementen geachtet werden, was hohe Ansprüche an das Geschick des Anwenders stellt.

WO2007/102156 A2, DE 81 36 484 U1 und FR 2 939 638A1 beschreiben verschiedene Nagelkorrekturvorrichtungen, die alle so konzipiert sind, dass beim Gebrauch ein Einschieben des zur Korrektur verwendeten Drahts (oder Spange) von vorne nicht möglich ist und somit ein bequemes und weitgehend schmerzfreies Anbringen der Korrekturvorrichtung nicht gewährleistet ist.

EP 2 158 881 A1 betrifft eine Nagelkorrekturvorrichtung, bei der ein auf den Nagel aufzuklebendes Haftelement auf der Oberseite mit Zapfen versehen ist, die in Aufbringen des Haftelements erleichtern sollen. Merkmale, die eine Einführung des Korrekturdrahts von vorne und dessen Befestigung durch Einrasten in eine Hinterschneidung ermöglichen, sind nicht erkennbar.

Aufgabe der vorliegenden Erfindung ist es, eine Befestigungsvorrichtung für verschiedenartige Nagelkorrekturspangen zu schaffen, welche einfach und schnell auf dem Nagel anbringbar ist und gleichzeitig die angebrachten Nagelkorrekturspangendrähte sicher und dauerhaft fixiert.

Im Laufe von Entwicklungsarbeiten im Rahmen der vorliegenden Erfindung wurde festgestellt, dass es von besonderem Vorteil ist, wenn die Befestigungsvorrichtung dergestalt ausführt ist, dass ein Haftelement fest mit einem Aufnahmeelement verbunden ist, welches neben seiner Hinterschneidung zur sicheren Aufnahme des Drahtes der Nagelkorrekturspange gleichzeitig als Manipulationshilfe beim Aufkleben des Heftelementes und Einführhilfe zum Anbringen des Nagelkorrekturspangenteils dient.

Der Gegenstand der Erfindung ist im Anspruch 1 definiert.

Fortentwicklungen und besondere Ausführungsformen der Erfindung ergeben sich aus den Unteransprüchen.

Nachstehend wird die Erfindung beispielhaft anhand einiger Zeichnungen näher erläutert:
Dabei zeigen:
Fig. 1 die erfindungsgemäße Befestigungsvorrichtung für Nagelkorrekturspangen im auf dem Nagel montierten Zustand mit eingehängten Nagelkorrekturdrähten;
Fig. 2 die erfindungsgemäße Befestigungsvorrichtung für Nagelkorrekturspangen im fertig auf dem Nagel montierten Zustand mit um Greifhilfe und Einführhilfe gekürztem Aufnahmeelement und gekürzten Nagelkorrekturdrähten;
Figg. 3 und 4 die erfindungsgemäße Befestigungsvorrichtung für Nagelkorrekturspangen im Lieferzustand in Seitenansicht und Draufsicht;
Fig. 5 eine alternative Ausführung des Aufnahmeelementes als Blechstreifen;
Fig. 6 eine alternative Ausführungsmöglichkeit der Manipulationshilfe zum einfacheren Greifen der Vorrichtung beim Aufkleben;
Fig. 7 ergänzende Befestigungselemente in Form von Widerhaken zur Fixierung des Nagelkorrekturspangendrahtes.

Nachstehend wird auf die in den Figuren dargestellten Ausführungsformen näher eingegangen.

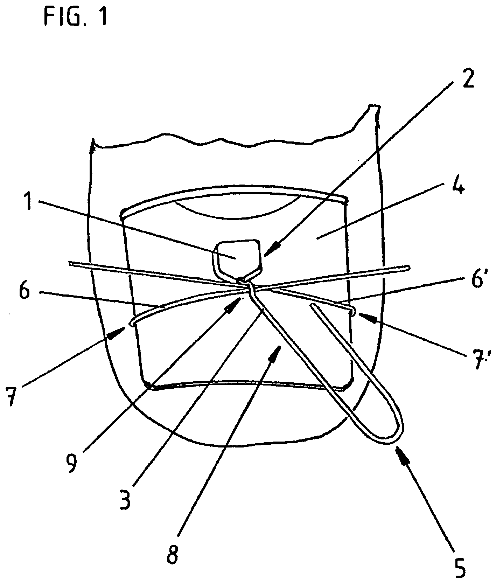

Figur 1 zeigt die erfindungsgemäße Befestigungsvorrichtung für Nagelkorrekturspangen im auf den Nagel montierten Zustand. Zur Behandlung des eingewachsenen Nagels 4 einer menschlichen Zehe sollen die Nagelränder minimal angehoben werden, um den schmerzenden Nagelfalz zu entlasten. Hierfür wird das Haftelement 1 der Befestigungsvorrichtung 2, welches fest mit dem, in einer bevorzugten Ausführung aus Draht bestehenden, Aufnahmeelement 3 verbunden ist, mit einem schnell aushärtenden Klebstoff auf die Nageloberfläche 4 aufgeklebt. Zur Handhabung während des Aufklebens dient die Greifhilfe, welche in einer bevorzugten Ausführung als aus dem Draht des Aufnahmeelementes 3 geformter Bügel 5 gestaltet ist, welcher sowohl mit den Fingern als auch mit einer Zange einfach festgehalten werden kann. Der Anpressdruck für die Klebung wird mittels Finger auf die Oberseite des Haftelementes 1 ausgeübt.

Nach dem Anbringen der Befestigungsvorrichtung 2 kann ein Nagelkorrekturspangenschenkel 6 aus Federdraht, welcher mit einem kleinen Häkchen 7 versehen ist, unter einen Rand des Nagels 4 eingehängt werden und von vorne entlang des als Einführhilfe entsprechend abgeschrägten Bereichs 8 des Aufnahmeelementes 3 in die Hinterschneidung 9 der Befestigungsvorrichtung 2 geschoben werden. Der Draht des Nagelkorrekturspangenschenkels 6 wird beim Einschieben unter das Aufnahmeelement 3 entsprechend der Krümmung des Nagels 4 elastisch verformt, woraus eine Rückstellkraft resultiert, welche einerseits den Rand des Nagels 4 leicht anhebt, andererseits den Nagelkorrekturspangenschenkel 6 in die Hinterschneidung 9 einrasten lässt und ihn dort dauerhaft fixiert.

Anschließend kann, soweit dies für den Therapieerfolg vorteilhaft ist, durch dasselbe Vorgehen ein zweiter, unter den gegenüberliegenden Rand des Nagels 4 eingehängter Nagelkorrekturspangenschenkel 6' angebracht werden.

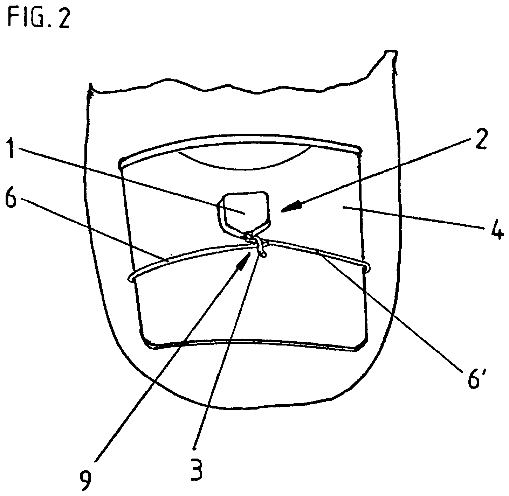

Nach dem Anbringen der Nagelkorrekturspangenschenkel 6,6' werden, wie in Figur 2 dargestellt, deren über das Aufnahmeelement 3 hinausragende Drahtenden auf zirka einen Millimeter gekürzt. Ebenso wird das Aufnahmeelement 3 der erfindungsgemäßen Befestigungsvorrichtung 2 soweit gekürzt, dass lediglich der Bereich der Hinterschneidung 9 am Haftelement 1 verbleibt. Zur Sicherung der Verbindung zwischen den einzelnen Teilen sowie zum Schutz vor Verletzungen ist es vorgesehen, den Bereich der abgetrennten Drähte sowie des gekürzten Aufnahmeelementes 3 mit einer Schutzschicht aus Kunststoff zu überziehen. Hierfür eignet sich beispielsweise UV-aushärtende Nagelprothetikmasse.

Alternativ zu der in den Figuren 1 und 2 beschriebenen Anwendung mit einem oder zwei unter den Nagelrand eingehängten Drahtschenkeln, kann die erfindungsgemäße Befestigungsvorrichtung gleichermaßen für Nagelkorrekturdrähte verwendet werden, welche nicht unter den Nagelrand eingehängt, sondern auf den Nagel aufgeklebt oder aber zwischen zwei der beschriebenen erfindungsgemäßen Befestigungsvorrichtungen, welche entsprechend an den Rändern des Nagels angebracht sind, gespannt werden.

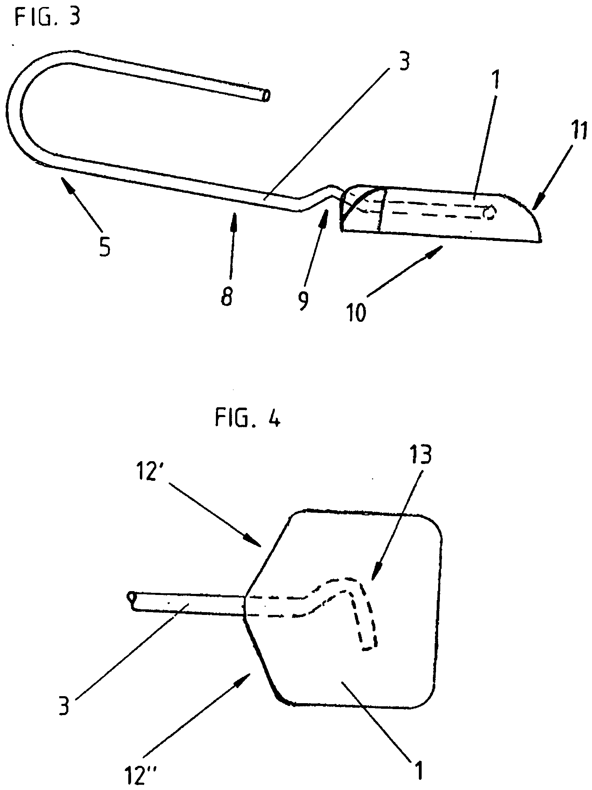

Figur 3 zeigt die Seitenansicht der erfindungsgemäßen Befestigungsvorrichtung im Lieferzustand. Die Befestigungsvorrichtung setzt sich aus einem Haftelement 1 sowie einem Aufnahmeelement 3 zusammen.

Das Haftelement 1, welches in einer bevorzugten Ausführung aus Kunststoff besteht, weist auf seiner Unterseite 10 eine Klebefläche auf, welche zur besseren Anlage auf der Nageloberfläche konkav gekrümmt sein kann. Die obere Kante 11 des Haftelementes 1 ist abgerundet, um ein Hängenbleiben mit den Strümpfen zu vermeiden und der Vorrichtung eine optisch flache und ansprechende Erscheinung zu geben.

In das Haftelement 1 ist ein Aufnahmeelement 3, welches in einer bevorzugten Ausführung aus einem Metalldraht besteht, fest eingebunden. Das Aufnahmeelement 3 weist eine Hinterschneidung 9 auf, welche zur Aufnahme der Nagelkorrekturspangenteile aus Draht dient. Bereits die dargestellte, relativ kleine Hinterschneidung reicht aus, um den Nagelkorrekturspangendraht sicher und dauerhaft zu halten.

Zusätzlich ist das Aufnahmeelement 3 dergestalt ausgeführt, dass es gleichzeitig als Einführhilfe für die Nagelkorrekturspangenteile in die Hinterschneidung dient, was mittels einer zur Unterseite 10 der Vorrichtung hinlaufende Schräge 8 realisiert werden kann, sowie durch die Ausformung als Drahtbügel 5 eine Greifhilfe für die einfache Handhabung der Befestigungsvorrichtung mit den Fingern oder mit Werkzeugen darstellt.

Figur 4 zeigt in der Draufsicht das Haftelement 1 sowie einen Teil des Aufnahmeelementes 3 der erfindungsgemäßen Befestigungsvorrichtung.

Die Kanten 12',12" der Vorderseite des Haftelementes 1 sind in einer bevorzugten Ausführung abgeschrägt ausgestaltet, um das Abschneiden der Nagelkorrekturspangenteile möglichst nahe am Aufnahmeelement 3 zu erleichtern, indem dadurch ausreichend Platz für die Spitze eines Seitenschneiders vorhanden ist.

Das in das Haftelement 1 eingebettete Ende des Aufnahmeelementes 3 ist in einer bevorzugten Ausführung zur besseren Verankerung und zur Sicherung gegen Verdrehen beispielsweise mit einer Abknickung 13 versehen.

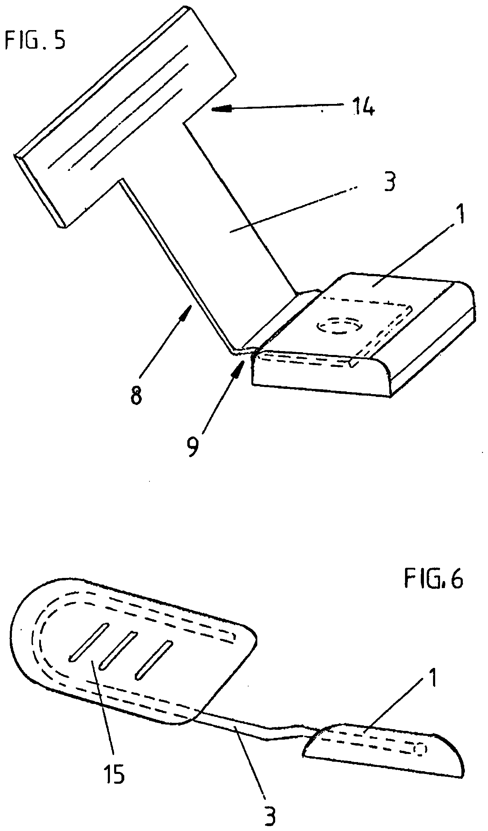

Wie in Figur 5 dargestellt, kann alternativ zur oben beschriebenen Ausführung das Aufnahmeelement 3 beispielsweise aus einem flachen Blechstreifen bestehen, welcher ebenfalls fest in das Haftelement 1 eingebettet ist. Auch dieses Aufnahmeelement 3 weist eine Hinterschneidung 9 zur Aufnahme des Drahtes der Nagelkorrekturspangenteile sowie die zum Einführen des Drahtes dienende Einführhilfe in Form einer zur Hinterschneidung 9 hinlaufenden Schräge 8 auf. Als Manipulationshilfe kann das Blech des Aufnahmeelementes 3 beispielsweise als Griff 14 geformt sein.

Bei der in Figur 6 dargestellten, beispielhaften Ausführung der erfindungsgemäßen Befestigungsvorrichtung ist an das als Draht ausgeführte Aufnahmeelement 3 ein Griff 15 angebracht, welcher die Handhabung der Befestigungsvorrichtung mit den Fingern deutlich erleichtert. Dieser Griff 15 ist vorzugsweise als ein angespritztes Kunststoffelement realisiert. Wie auch bei den vorgenannten Ausführungsformen wird diese Manipulationshilfe nach der vollendeten Montage abgetrennt.

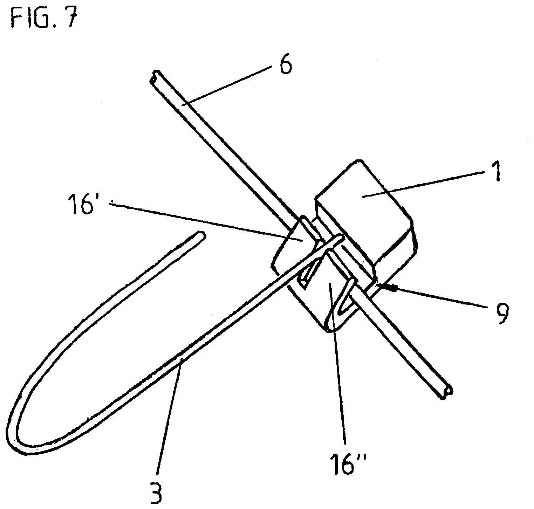

Figur 7 zeigt beispielhaft ergänzende Elemente zur dauerhaften Fixierung der Nagelkorrekturspangenteile in der erfindungsgemäßen Befestigungsvorrichtung.

In der dargestellten Ausführung weist die Befestigungsvorrichtung zwei am Haftelement 1 angebrachte, flexible Widerhaken 16',16" auf, welche den unter das Aufnahmeelement 3 eingeführten Nagelkorrekturspangendraht 6 zusätzlich fixieren.

Die erfindungsgemäße Befestigungsvorrichtung für Nagelkorrekturspangen erweist sich in vieler Hinsicht als vorteilhaft, da sie eine sehr einfache Möglichkeit bietet, verschiedenste bekannte Nagelkorrektursysteme flexibel und für den jeweiligen Fall optimal miteinander zu kombinieren.

So besteht die Möglichkeit, einseitige oder beidseitige Nagelkorrekturen durchzuführen, sowie gleichzeitig zwischen unter den Nagelrand eingehängten als auch auf den Nagel aufgeklebten Nagelkorrekturspangenteilen zu variieren. Dies stellt einen besonderen Vorteil dar, da oft erst während der Anwendung entschieden werden kann, ob es möglich ist, ein Drahthäkchen unter den Nagelrand einzubringen.

Das Aufkleben der Vorrichtung ist auf Grund der Manipulationshilfe am Aufnahmeelement und der Möglichkeit, mit dem Finger einen großen Anpressdruck auf das Haftelement auszuüben, besonders einfach. Ebenso ist das Einführen der Nagelkorrekturspangenteile auf Grund der Formgebung des Aufnahmeelementes auch für ungeübte Anwender sehr einfach und lässt sich, beispielsweise wenn das Häkchen des Nagelkorrekturspangenteiles wieder unter dem Nagelrand herausspringt, beliebig oft wiederholen. Dies stellt einen Vorteil gegenüber anderen aufklebbaren Spangen dar, da diese oft unter Spannung aufgeklebt und teilweise zusätzlich zeitgleich unter den Nagelrand eingehakt werden müssen.

Auf Grund der genannten Vorteile werden Nagelkorrekturspangen mit dieser Befestigungsvorrichtung auch der Anforderung der Wirtschaftlichkeit in der ärztlichen, podologischen und kosmetischen Praxis besonders gerecht.

Für den Patienten erweist sich neben der optimalen Behandlung seiner Beschwerden, die besonders flache Bauweise der Befestigungsvorrichtung als vorteilhaft. Sie wird auch in engen Schuhen nicht als störend empfunden und bietet dem Patienten damit einen hohen Tragekomfort.

## Patentansprüche

1. Befestigungsvorrichtung für Nagelkorrekturspangen, umfassend:
a) ein Haftelement (1), das auf die Nagelplatte (4) aufklebbar ist,
b) ein fest am Haftelement (1) angebrachtes Aufnahmeelement (3) zum Aufnehmen eines Nagelkorrekturspangenteils aus Draht, wobei das Aufnahmeelement (3) eine Hinterschneidung (9) zur formschlüssigen Fixierung des beim Gebrauch von vorne einschiebbaren Nagelkorrekturspangenteils (6) aufweist und wobei das Aufnahmeelement (3) so ausgeformt ist, dass es gleichzeitig als Einführhilfe für den Draht des Nagelkorrekturspangenteils (6) dient und eine Greifmöglichkeit (5) zur Manipulation beim Aufkleben des Haftelements (1) bietet,
**dadurch gekennzeichnet, dass** das Aufnahmeelement (3) aus Draht besteht.

2. Befestigungsvorrichtung für Nagelkorrekturspangen nach Anspruch 1, **dadurch gekennzeichnet, dass** das Aufnahmeelement (3) aus gebogenem Blech besteht.

3. Befestigungsvorrichtung für Nagelkorrekturspangen nach einem der vorstehenden Ansprüche, **dadurch gekennzeichnet, dass** das Haftelement (1) aus Kunststoff besteht.

4. Befestigungsvorrichtung für Nagelkorrekturspangen nach einem der vorstehenden Ansprüche, **dadurch gekennzeichnet, dass** das Haftelement (1) abgeschrägte Flanken (12', 12") zur Erleichterung des Abschneidens eingehängter Nagelkorrekturspangenteile (6,6') aufweist.

5. Befestigungsvorrichtung für Nagelkorrekturspangen nach einem der vorstehenden Ansprüche, **dadurch gekennzeichnet, dass** die Greifmöglichkeit zur Manipulation durch eine Formung des Aufnahmeelementes (3) als Bügel (5) realisiert ist.

6. Befestigungsvorrichtung für Nagelkorrekturspangen nach einem der Ansprüche 1 bis 4, **dadurch gekennzeichnet, dass** die Greifmöglichkeit zur Manipulation durch ein angespritztes Kunststoffelement (15) realisiert ist.

7. Befestigungsvorrichtung für Nagelkorrekturspangen nach einem der Ansprüche 1 bis 6, **dadurch gekennzeichnet, dass** das Grundmaterial des Aufnahmeelementes (3) mit Kunststoff ummantelt ist.

8. Befestigungsvorrichtung für Nagelkorrekturspangen nach einem der vorstehenden Ansprüche, **dadurch gekennzeichnet, dass** die Unterseite des Haftelementes (1) zur verbesserten Klebung zur Anlage auf der Nageloberfläche (4) konkav gekrümmt ist.

## Claims

1. Fastening device for nail correcting braces comprising:
a) a bonding member (1) adapted to be adhered on the nail body (4),
b) a receiving member (3) fixedly attached to the bonding member (1) for receiving a nail correcting brace part of wire, the receiving member (3) having an undercut (9) for form-locking fixing of the nail correcting brace part (6) insertable from the front in use and the receiving member (3) being so formed that it simultaneously serves as insertion aid for the wire of the nail correcting brace part (6) and offers a grip facility (5) for manipulation in the adhering of the bonding member (1),
**characterized in that** the receiving member (3) consists of wire.

2. Fastening device for nail correcting braces according to claim 1, **characterized in that** the receiving member (3) consists of curved sheet metal.

3. Fastening device for nail correcting braces according to any one of the preceding claims, **characterized in that** the bonding member (1) consists of plastic.

4. Fastening device for nail correcting braces according to any one of the preceding claims, **characterized in that** the bonding member (1) has bevelled edges (12', 12") for facilitating cutting off suspended nail correcting brace parts (6, 6').

5. Fastening device for nail correcting braces according to any one of the preceding claims, **characterized in that** the grip facility for manipulation is implemented by a configuration of the receiving member (3) as stirrup (5).

6. Fastening device for nail correcting braces according to any one of claims 1 to 4, **characterized in that** the grip facility for manipulation is implemented by a plastic member (15) injection moulded thereon.

7. Fastening device for nail correcting braces according to any one of claims 1 to 6, **characterized in that** the basic material of the receiving member (3) is covered with plastic.

8. Fastening device for nail correcting braces according to any one of the preceding claims, **characterized in that** the bottom side of the bonding member (1) is concavely curved for improved adhesion for attachment to the nail surface (4).

## Revendications

1. Dispositif de fixation pour agrafes de correction d'ongle, comprenant :
a) un élément collant (1) qui peut être collé sur la plaque de l'ongle (4),
b) un élément de réception (3) appliqué fixement sur l'élément collant (1) pour recevoir une partie d'agrafe de correction d'ongle faite d'un fil, dans lequel l'élément de réception (3) présente une contre-dépouille (9) pour une fixation par complémentarité de formes de la partie d'agrafe de correction d'ongle (6) pouvant être insérée par l'avant lors de l'utilisation et dans lequel l'élément de réception (3) est ainsi formé qu'il sert parallèlement d'aide à l'introduction pour le fil de la partie d'agrafe de correction d'ongle (6) et offre une possibilité de préhension (5) pour la manipulation lors du collage de l'élément collant (1),
**caractérisé en ce que** l'élément de réception (3) est fait d'un fil.

2. Dispositif de fixation pour agrafes de correction d'ongle selon la revendication 1, **caractérisé en ce que** l'élément de réception (3) est en tôle incurvée.

3. Dispositif de fixation pour agrafes de correction d'ongle selon l'une des revendications précédentes, **caractérisé en ce que** l'élément collant (1) est en plastique.

4. Dispositif de fixation pour agrafes de correction d'ongle selon l'une des revendications précédentes, **caractérisé en ce que** l'élément collant (1) présente des flancs en biais (12', 12") pour faciliter la coupe des parties d'agrafe de correction d'ongle en fil (6, 6') accrochées.

5. Dispositif de fixation pour agrafes de correction d'ongle selon l'une des revendications précédentes, **caractérisé en ce que** la possibilité de préhension pour la manipulation est réalisée par une formation de l'élément de réception (3) en tant qu'étrier (5).

6. Dispositif de fixation pour agrafes de correction d'ongle selon l'une des revendications 1 à 4, **caractérisé en ce que** la possibilité de préhension pour la manipulation est réalisée par un élément plastique (15) injecté.

7. Dispositif de fixation pour agrafes de correction d'ongle selon l'une des revendications 1 à 6, **caractérisé en ce que** le matériau de base de l'élément de réception (3) est enveloppé de plastique.

8. Dispositif de fixation pour agrafes de correction d'ongle selon l'une des revendications précédentes, **caractérisé en ce que** la sous-face de l'élément collant (1) est incurvée de manière concave pour un collage amélioré pour le placement sur la surface de l'ongle (4).
